# DEMANDE DE BREVET EUROPEEN

(11) **EP 4 299 016 A1**
(43) Date de publication de la demande: **03.01.2024**
(21) Numéro de dépôt: 23179939.6
(22) Date de dépôt: 19.06.2023
(51) Int. Cl.: A61B 8/12, A61B 8/00

(54) **DISPOSITIF MEDICAL IMPLANTABLE ET PROCEDE DE FABRICATION D'UN TEL DISPOSITIF**

(30) Priorité: 28.06.2022 FR 2206419
(71) Demandeur: Vermon, 37000 Tours (FR)
(72) Inventeur: HOANG, Thien, 37000 TOURS (FR); ROSINSKI, Bogdan, 37000 TOURS (FR); FELIX, Nicolas, 37000 TOURS (FR)
(74) Mandataire: Cabinet Beaumont

(57) **Abrégé**

La présente description concerne un dispositif médical implantable comportant un premier substrat (101) en un matériau biocompatible, le premier substrat (101) définissant une partie d'un boîtier du dispositif et présentant une face externe destinée à être mise en contact avec des tissus biologiques d'un utilisateur, le dispositif comportant au moins un transducteur ultrasonore (107) disposé dans une cavité (103) formée du côté d'une face interne du premier substrat (101), le transducteur (107) étant agencé de sorte qu'aucune couche d'air, de gaz ou de vide ne sépare le transducteur (107) du premier substrat (101) .

## Description

### Domaine technique

La présente description concerne de façon générale les dispositifs et systèmes à base de transducteurs ultrasonores, et vise plus particulièrement un dispositif médical implantable intégrant un ou plusieurs transducteurs ultrasonores, et un procédé de fabrication d'un tel dispositif.

### Technique antérieure

Un dispositif médical implantable est un dispositif destiné à être implanté dans le corps d'un patient pour surveiller et/ou traiter divers types de pathologies. Un tel dispositif peut comprendre un ou plusieurs capteurs, par exemple pour mesurer un ou plusieurs paramètres physiologiques, et/ou un ou plusieurs actionneurs, par exemple pour administrer un traitement ou stimuler un organe.

Ces éléments sont généralement logés dans un boîtier en un matériau biocompatible, que l'on vient implanter dans le corps du patient.

Un dispositif médical implantable comprend classiquement une batterie électrique non rechargeable pour alimenter ses différents composants. Ceci présente toutefois des limitations en termes de durée de vie et d'encombrement.

Il serait souhaitable d'améliorer au moins en partie certains aspects des dispositifs médicaux implantables connus.

Pour réduire l'encombrement et/ou augmenter la durée de vie d'un dispositif médical implantable, il a été proposé d'utiliser une batterie rechargeable alimentée par un système de transfert d'énergie sans fil, et, plus particulièrement, par un système de transfert d'énergie sans fil par ultrasons.

La présente demande vise une intégration avantageuse d'un ou plusieurs transducteurs ultrasonores dans un dispositif médical implantable, notamment pour des applications de recharge électrique sans fil par ultrasons.

### Résumé de l'invention

Selon un aspect d'un mode de réalisation, pour réduire l'encombrement et/ou augmenter la durée de vie d'un dispositif médical implantable, on prévoit d'utiliser une batterie rechargeable alimentée par un système de transfert d'énergie sans fil, et, plus particulièrement, par un système de transfert d'énergie sans fil par ultrasons.

La présente description vise une intégration avantageuse d'un ou plusieurs transducteurs ultrasonores dans un dispositif médical implantable, notamment pour des applications de recharge électrique sans fil par ultrasons.

Un mode de réalisation prévoit un dispositif médical implantable comportant un premier substrat en un matériau biocompatible, le premier substrat définissant une partie d'un boîtier du dispositif et présentant une face externe destinée à être mise en contact avec des tissus biologiques d'un utilisateur, le dispositif comportant au moins un transducteur ultrasonore disposé dans une cavité formée du côté d'une face interne du premier substrat, le transducteur étant agencé de sorte qu'aucune couche d'air, de gaz ou de vide ne sépare le transducteur du premier substrat.

Selon un mode de réalisation, la cavité est une cavité non traversante formée dans le premier substrat, du côté de la face interne du premier substrat.

Selon un mode de réalisation, le premier substrat est en un matériau électriquement isolant, par exemple du saphir.

Selon un mode de réalisation, le dispositif comprend en outre un deuxième substrat fixé sur le premier substrat, du côté de la face interne du premier substrat, la cavité étant une cavité traversante formée dans le deuxième substrat et débouchant sur le premier substrat.

Selon un mode de réalisation, le premier substrat est en métal, par exemple en titane.

Selon un mode de réalisation, le deuxième substrat est en un matériau électriquement isolant.

Selon un mode de réalisation, le deuxième substrat comprend une carte de circuit imprimé.

Selon un mode de réalisation, le dispositif comporte en outre, au-dessus de la face supérieure du premier substrat, des éléments métalliques d'interconnexion connectés respectivement à des première et deuxième électrodes du transducteur.

Selon un mode de réalisation, le dispositif comporte en outre, au-dessus de la face supérieure du premier substrat, un circuit électronique de contrôle connecté aux première et deuxième électrodes du transducteur par l'intermédiaire des éléments métalliques d'interconnexion.

Selon un mode de réalisation, le dispositif comporte en outre une batterie électrique au-dessus de la face supérieure du premier substrat et connecté au circuit électronique de contrôle.

Selon un mode de réalisation, la batterie est disposée dans une cavité formée du côté de la face interne du premier substrat, à l'intérieur du boîtier.

Selon un mode de réalisation, le circuit électronique de contrôle est adapté à convertir une puissance électrique fournie par le transducteur ultrasonore en un signal électrique de recharge de la batterie et/ou d'alimentation électrique de composants électroniques du dispositif.

Un autre mode de réalisation prévoit un procédé de fabrication d'un dispositif tel que défini ci-dessus, comprenant une étape de formation de la cavité, suivie d'une étape de report et de fixation du transducteur dans la cavité.

Selon un mode de réalisation, la cavité est formée par ablation laser.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

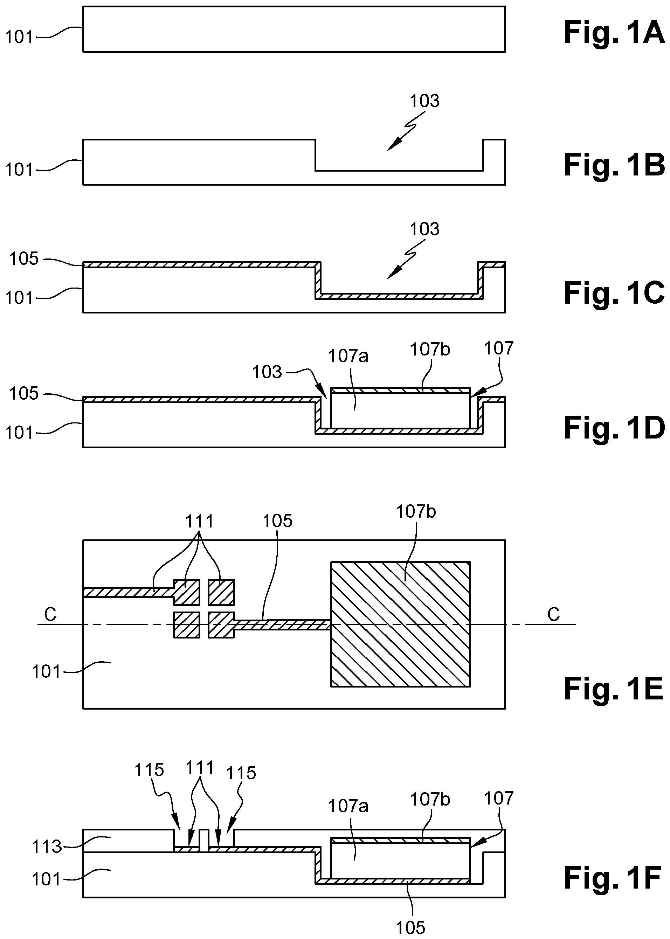

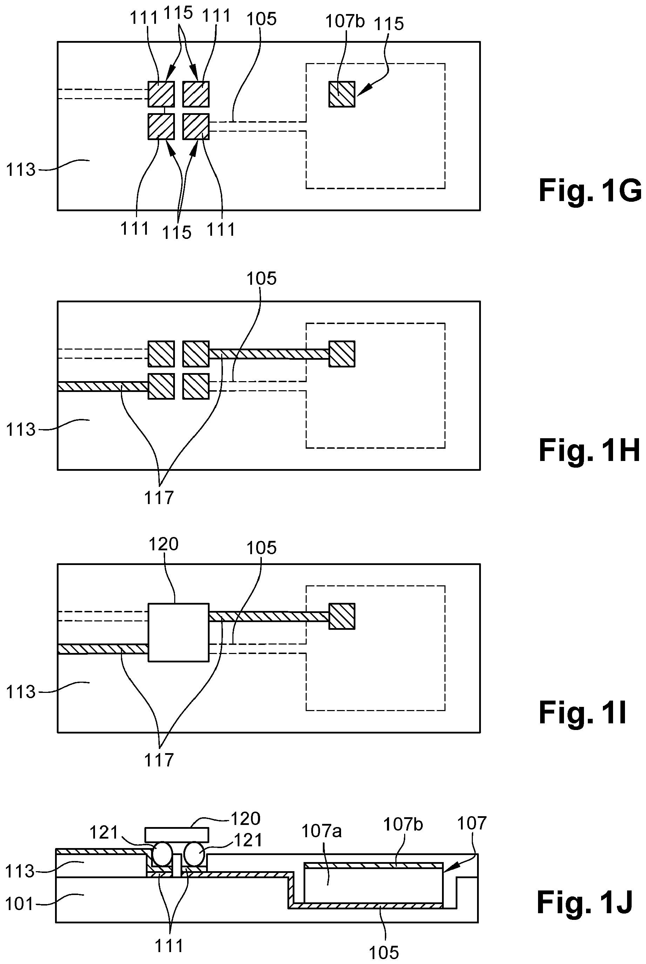

la figure 1A, la figure 1B, la figure 1C, la figure 1D, la figure 1E, la figure 1F, la figure 1G, la figure 1H, la figure 1I, et la figure 1J illustrent de façon schématique et partielle des étapes successives d'un exemple d'un mode de réalisation d'un procédé de fabrication d'un dispositif médical implantable comprenant un transducteur ultrasonore ;

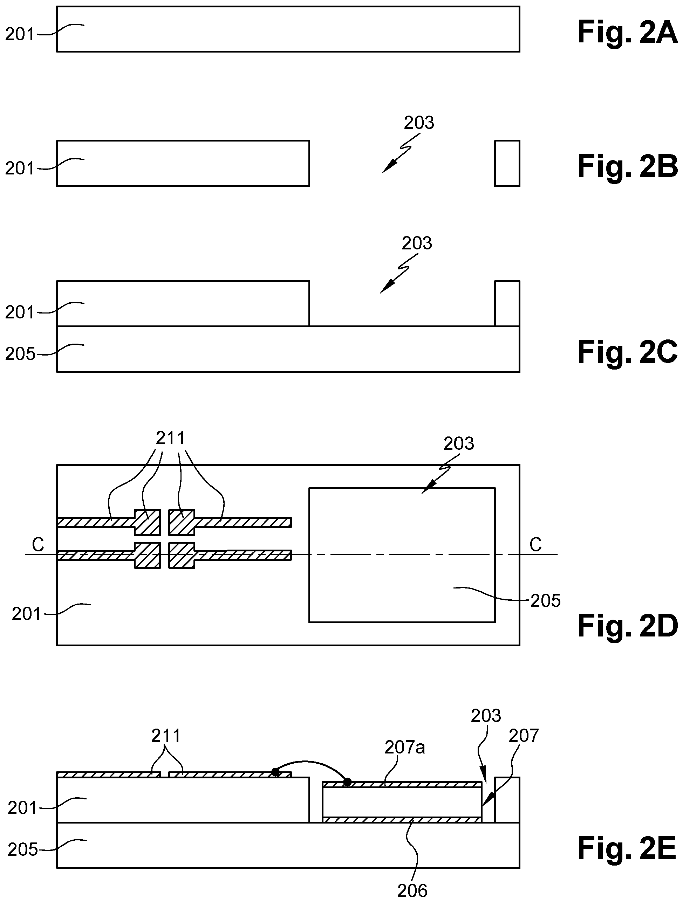

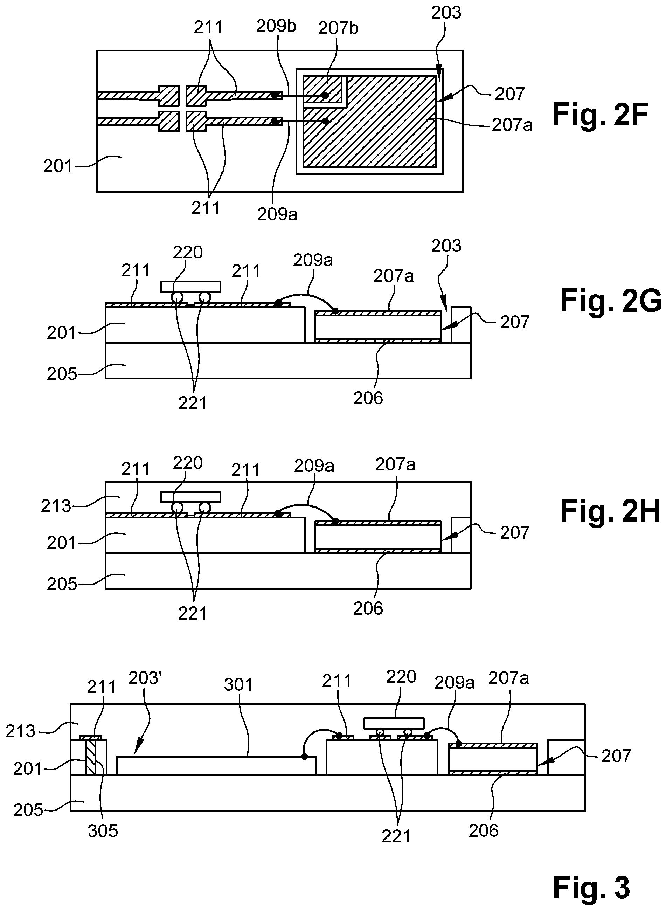

la figure 2A, la figure 2B, la figure 2C, la figure 2D, la figure 2E, la figure 2F, la figure 2G et la figure 2H illustrent de façon schématique et partielle des étapes successives d'un exemple d'un autre mode de réalisation d'un procédé de fabrication d'un dispositif médical implantable comprenant un transducteur ultrasonore ; et
la figure 3 illustre de façon schématique et partielle une variante du procédé des figures 2A à 2H.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, seule l'intégration d'un ou plusieurs transducteurs ultrasonores au sein d'un dispositif médical implantable a été détaillée. La réalisation des transducteurs ultrasonores n'a pas été détaillée, les modes de réalisation décrits étant compatibles tous ou la plupart des transducteurs ultrasonores connus. En outre, la réalisation et l'intégration des autres éléments du dispositif médical implantable (batterie, capteurs, actionneurs, circuits électroniques de contrôle et/ou de traitement, etc.) n'a pas été détaillée, les modes de réalisation décrits étant compatibles avec les réalisations et intégrations usuelles de ces éléments ou la réalisation et l'intégration de ces éléments étant à la portée de la personne du métier à partir des indications de la présente description.

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des vues en coupe des figures.

Sauf précision contraire, les expressions "environ", "approximativement", "sensiblement", et "de l'ordre de" signifient à 10 % près, de préférence à 5 % près.

Les figures 1A à 1J illustrent de façon schématique et partielle des étapes successives d'un exemple d'un procédé de fabrication d'un dispositif médical implantable comprenant au moins un transducteur ultrasonore selon un mode de réalisation. Le transducteur est par exemple destiné à être utilisé pour des applications transfert d'énergie sans fil par ultrasons, par exemple pour recharger une batterie électrique (non détaillée sur les figures), du dispositif. Pour cela, un transducteur ultrasonore externe au dispositif médical implantable et alimenté par une source d'énergie électrique externe, émet des ultrasons en direction du transducteur ultrasonore du dispositif médical implantable. Le transducteur du dispositif médical implantable convertit les ultrasons reçus en énergie électrique pour recharger la batterie électrique du dispositif médical implantable. On réalise ainsi un transfert d'énergie électrique sans fil, par ultrasons, entre la source d'énergie électrique externe et la batterie électrique du dispositif médical implantable. A titre de variante ou de façon complémentaire, le ou les transducteurs ultrasonores du dispositif peuvent être utilisés pour des applications de communication avec un dispositif externe, ou pour des applications de détection (« sensing ») ou de mesure.

La figure 1A est une vue en coupe illustrant un substrat 101 biocompatible, par exemple électriquement isolant. Le substrat 101 est par exemple en saphir. A titre de variante (non détaillée sur les figures), le substrat peut être comprendre un support en un matériau non nécessairement biocompatible, revêtu par une couche ou enveloppe externe en un matériau biocompatible, par exemple un matériau polymère biocompatible, par exemple du parylène.

Le substrat 101 est par exemple destiné à former une partie d'un boîtier d'encapsulation du dispositif médical implantable. Dans cet exemple, la face supérieure du substrat 101 correspond à une face interne du boîtier, destinée à recevoir des composants internes du dispositif médical, et notamment au moins un transducteur ultrasonore. A titre d'exemple, la face inférieure du substrat 101 correspond à une face externe du boîtier, destinée à être mise en contact avec des tissus biologiques d'un patient. Le substrat 101 a par exemple la forme d'une plaque, par exemple rectangulaire, d'épaisseur sensiblement homogène. L'épaisseur du substrat 101 est par exemple comprise entre 500 µm et 3 mm. Les dimensions latérales de la plaque (correspondant aux dimensions latérales du boîtier) sont par exemple comprises entre 1 et 10 centimètres. Sur les figures 1A à 1J, la réalisation d'un unique dispositif médical implantable est (partiellement) représentée. En pratique, plusieurs dispositifs peuvent être formés en parallèle et de façon simultanée à partir d'un même substrat 101 de plus grandes dimensions latérales, puis singularisés lors d'une étape de découpe du substrat 101.

La figure 1B est une vue en coupe illustrant la structure obtenue à l'issue d'une étape de formation d'une cavité 103 localisée dans une partie de l'épaisseur du substrat 101. La cavité 103 s'étend verticalement depuis la face supérieure du substrat 101, sur une partie seulement de l'épaisseur du substrat. A titre d'exemple, la profondeur de la cavité 103 est comprise entre 0,1 et 2 mm. La cavité 103 est destinée à recevoir un transducteur ultrasonore du dispositif. A titre d'exemple, la cavité 103 présente des dimensions latérales comprises entre 0,2 et 2 cm, par exemple entre 0,5 et 1 cm. La cavité 103 est par exemple formée par ablation laser. En vue de dessus, la cavité 103 a par exemple une forme rectangulaire.

La figure 1C est une vue en coupe illustrant la structure obtenue à l'issue d'une étape de dépôt d'une couche métallique 105 sur la face supérieure du substrat 101. La couche métallique 105 s'étend par exemple de façon continue et avec une épaisseur sensiblement uniforme sur toute la surface supérieure du substrat 101, c'est à dire sur les flancs et au fond de la cavité 103, et sur la face supérieure du substrat 101 en dehors de la cavité 103. La couche métallique 105 est par exemple en contact, par sa face inférieure, avec la face supérieure du substrat 101. La couche métallique 105 est par exemple en or, en argent ou en cuivre. La couche métallique 105 peut comprendre un empilement (non détaillé sur les figures) de plusieurs couches de métaux différents. Par exemple, la couche métallique 105 peut comprendre une couche d'accroche, par exemple en nickel, en contact avec le substrat 101, surmontée d'une couche en un autre métal, par exemple de l'or, de l'argent ou du cuivre. La couche métallique 105 est par exemple déposée par un procédé de dépôt en phase vapeur (PVD - de l'anglais Phase Vapor Déposition). L'épaisseur de la couche métallique 105 est par exemple comprise entre 0,1 et 1 µm.

La figure 1D est une vue en coupe illustrant la structure obtenue à l'issue d'une étape de report et de fixation d'un transducteur ultrasonore 107 dans la cavité 103. Le transducteur 107 est par exemple un transducteur piézoélectrique. A titre d'exemple, le transducteur comprend une couche ou pastille 107a en un matériau piézoélectrique, et une électrode supérieure conductrice 107b, par exemple métallique, sur et en contact avec la face supérieure de la couche piézoélectrique 107a. La couche piézoélectrique 107 est par exemple en une céramique piézoélectrique, par exemple de type PZT (titano-zirconate de plomb). L'électrode 107b s'étend par exemple sur sensiblement toute la surface supérieure de la couche piézoélectrique 107a. Dans cet exemple, la couche piézoélectrique est fixée sur et connectée électriquement à la face supérieure de la couche métallique 105, au fond de la cavité 103, par exemple au moyen d'une colle, par exemple électriquement conductrice. Dans ce cas, la portion de la couche métallique 105 située au fond de la cavité 103 définit l'électrode inférieure du transducteur 107. A titre d'exemple, le transducteur 107 présente des dimensions latérales sensiblement identiques à celles de la cavité 103, par exemple inférieures de moins de dix pourcents aux dimensions latérales de la cavité 103. Ainsi, le transducteur 107 occupe sensiblement toute la surface de la cavité 103. L'épaisseur du transducteur 107 est par exemple sensiblement égale à la profondeur de la cavité 103, par exemple égale, à 20 pourcents près, de préférence à 10 pourcents près, à la profondeur de la cavité 103. Le transducteur 107 peut être prélevé sur un support et placé dans la cavité 103 au moyen d'un outil de prélèvement et placement (« pick and place » en anglais). Une ou plusieurs couches intermédiaires (non détaillées sur les figures) de réflexion (miroir acoustique) ou d'absorption des ondes acoustiques, par exemple électriquement conductrices, peuvent éventuellement être prévues entre la face supérieure du matériau piézoélectrique 107a et l'électrode supérieure 107b.

On notera que les modes de réalisation décrits ne se limitent pas à l'exemple décrit ci-dessus dans lequel le transducteur 107 est un transducteur piézoélectrique. Plus généralement, les modes de réalisations peuvent être adaptés à tout type de transducteur ultrasonore, par exemple des transducteurs capacitifs à membrane (CMUT), des transducteurs piézoélectriques à membrane (PMUT), etc. Une ou plusieurs couches intermédiaires d'adaptation d'impédance acoustique et/ou d'adaptation de travail de sortie, par exemple électriquement conductrices, peuvent éventuellement être prévues entre la face inférieure du transducteur et la couche métallique 105. En tout état de cause, selon un aspect du mode de réalisation des figures 1A à 1J, aucune couche d'air, de gaz ou de vide ne sépare la face inférieure du transducteur 107 de la face supérieure du substrat 101, au fond de la cavité 103. Autrement dit, le transducteur 107 est en contact mécaniquement avec la face supérieure du substrat 101, ou séparée de la face supérieure du substrat 101 uniquement par un ou plusieurs matériaux solides. Plus particulièrement, dans l'exemple détaillé en relation avec les figures, aucune couche d'air, de gaz ou de vide ne sépare la face inférieure de la couche piézoélectrique 107a du transducteur 107 de la face supérieure du substrat 101, au fond de la cavité 103. Autrement dit, la couche piézoélectrique 107a du transducteur est en contact mécaniquement avec la face supérieure du substrat 101, ou séparée de la face supérieure du substrat 101 uniquement par un ou plusieurs matériaux solides. Il en résulte, avantageusement, que la puissance acoustique reçue sur la face inférieure du substrat 101 en provenance d'un dispositif extérieur est transmise efficacement, avec peu de pertes, au transducteur 107. A l'inverse, en cas d'émission d'ondes ultrasonores par le transducteur 107, la puissance acoustique émise par le transducteur est transmise efficacement et avec peu de pertes vers l'extérieur du dispositif, par l'intermédiaire du substrat 101.

Dans le mode de réalisation des figures 1A à 1J, l'amincissement localisé du substrat 101 en vis à vis du transducteur 107 (cavité 103) présente le double avantage de réduire l'encombrement global du dispositif, et de permettre une meilleure transmission des ondes acoustiques entre le transducteur et l'extérieur du boîtier. De préférence, l'épaisseur du substrat 101 subsistant au fond de la cavité 103 est choisie de façon à favoriser le couplage acoustique entre le transducteur 107 et le milieu extérieur au boîtier. A titre d'exemple, l'épaisseur du substrat 101 subsistant au fond de la cavité 103 est comprise entre 0,2 et 1 mm. La fixation du transducteur 107 dans la cavité 103 permet de préserver la rigidité du boîtier à l'emplacement de la cavité.

La figure 1E est une vue de dessus illustrant la structure obtenue à l'issue d'une étape de retrait localisé de la couche métallique 105 sur la face supérieure du substrat 101, par exemple par photolithographie et gravure ou par ablation laser, de façon à définir des pistes conductrices et plages conductrice d'interconnexion 111 du dispositif. On notera que les vues en coupe des figures 1A à 1J sont prises dans le plan de coupe C-C de la figure 1E.

A titre de variante, les étapes des figures 1D (report et fixation du transducteur 107 dans la cavité 103) et 1E (gravure des éléments d'interconnexion 111 dans la couche métallique 105) peuvent être inversées, c'est à dire que les étapes de la figure 1E peuvent être mises en oeuvre avant les étapes de la figure 1D.

La figure 1F est une vue en coupe illustrant la structure obtenue à l'issue d'une étape de dépôt d'une couche électriquement isolante de passivation 113 sur la face supérieure de la structure de la figure 1E, suivie d'une étape de formation d'ouvertures localisées 115 dans la couche 113.

La couche 113 est par exemple une couche en un matériau polymère, par exemple un polyimide. La couche 113 est par exemple d'abord déposée sur toute la surface supérieure de la structure de la figure 1E. L'épaisseur de la couche 113 est par exemple comprise entre 10 et 100 µm.

La couche 113 est ensuite localement retirée, par exemple par ablation laser, de façon à former des ouvertures traversantes 115 à l'aplomb de plages métalliques de connexion 111 préalablement définies dans la couche métallique 105 (figure 1D). Les ouvertures 115 débouchent sur la face supérieure des plages métalliques de connexion 111 afin de permettre la reprise ultérieure d'un contact électrique sur les plages de connexion 111. Une ou plusieurs ouvertures localisées de reprise de contact 115 (non visibles sur la figure 1F) peuvent en outre être formées en vis à vis de l'électrode supérieure 107b du transducteur 107, de façon à permettre la reprise d'un contact électrique sur l'électrode 107b.

La figure 1G est une vue de dessus de la structure de la figure 1F. Dans cet exemple, quatre ouvertures localisées 115 ont été formées en vis à vis respectivement de quatre plages de connexion métalliques 111 disposées sur la face supérieure du substrat 101. L'une de ces plages métalliques 111 est connectée électriquement à l'électrode inférieure du transducteur 107 par l'intermédiaire d'une piste conductrice définie dans le niveau métallique 105. Les plages métalliques 111 sont par exemple destinées à être connectées à des plots de connexion correspondants d'un circuit électronique de contrôle du transducteur, par exemple un circuit électronique intégré dans et sur un substrat semiconducteur, par exemple un circuit CMOS. Dans cet exemple, une ouverture localisée 115 supplémentaire est en outre formée en vis à vis de l'électrode supérieure 107b du transducteur.

La figure 1H est une vue de dessus illustrant la structure obtenue à l'issue d'une étape de formation d'éléments métalliques d'interconnexion 117 sur la face supérieure de la structure de la figure 1G. A titre d'exemple, une couche métallique s'étendant de façon continue sur toute la surface supérieure de la structure peut d'abord être déposée, cette couche étant ensuite retirée localement, par exemple par photolithographie et gravure, pour définir les éléments d'interconnexion 117. A titre d'exemple, parmi les éléments d'interconnexion 117, une piste conductrice connecte électriquement la portion de l'électrode supérieure 107b du transducteur exposée à l'étape précédente à l'une des plages métalliques 111 exposée également à l'étape précédente.

Les figure 1I et 1J sont respectivement une vue de dessus et une vue en coupe de la structure obtenue à l'issue d'une étape de report d'un circuit électronique de contrôle 120 sur la face supérieure de la structure de la figure 1H.

Dans cet exemple, le circuit électronique de contrôle 120 est un circuit intégré dans et sur un substrat semiconducteur, par exemple un substrat de silicium. Le circuit 120 présente des plots de connexion 121 connectés électriquement, par exemple par soudure ou de brasure, à des plages métalliques de connexion correspondante du dispositif, formées, par exemple, dans le niveau métallique 105/111, et/ou dans le niveau métallique 117. Dans cet exemple, le circuit électronique de contrôle comprend deux plots de connexion connectés respectivement à l'électrode inférieure et à l'électrode supérieure du transducteur 107. Le circuit électronique de contrôle 120 peut en outre comprendre d'autres plots de connexion connectés à des bornes d'alimentation et/ou d'entrée/sortie de signaux de contrôle et/ou de données du dispositif. A titre de variante, le circuit électronique de contrôle 120 peut comprendre plusieurs puces semiconductrices et/ou plusieurs composants électroniques discrets interconnectés entre eux et avec le transducteur ultrasonore 107 par l'intermédiaire d'éléments d'interconnexion du niveau métallique 105/111, et/ou du niveau métallique 117. Les niveaux métalliques 105/111 et 117 peuvent être utilisés pour interconnecter d'autres éléments (non détaillé) du dispositif médical. En pratique, le nombre de nivaux métalliques d'interconnexion formés sur la face supérieure du substrat 101 peut être différent de deux, par exemple égal à 1 ou supérieur à 2.

Par ailleurs, bien que non représentée sur les figures, une batterie électrique peut être reportée sur la face supérieure du substrat 101 et connectée électriquement à des éléments d'interconnexion formés sur la face supérieure du substrat 101. La batterie électrique est par exemple connectée électriquement au circuit électronique de contrôle 120. A titre d'exemple, le circuit électronique 120 comprend un circuit de conversion de puissance adapté à recharger la batterie électrique au moyen de l'énergie électrique générée aux bornes du transducteur ultrasonore sous l'effet d'une onde acoustique en provenance d'un dispositif externe.

Pour limiter l'encombrement, la batterie peut-être entièrement ou partiellement logée dans une cavité préalablement formée dans le substrat 101, par exemple une cavité similaire à la cavité 103 mais présentant des dimensions adaptées aux dimensions de la batterie.

A l'issue de ces étapes, un capot supérieur (non détaillé sur les figures) du boîtier du dispositif, de préférence en un matériau biocompatible, par exemple en le même matériau que le substrat 101, peut être rapporté sur la face supérieure de la structure, de façon à encapsuler hermétiquement les composants internes du dispositif. A titre d'exemple, le capot supérieur est soudé à la partie inférieure du boîtier, formée par le substrat 101, par exemple par soudure laser. A titre d'exemple, le boîtier formé par le substrat 101 et le capot supérieur encapsule hermétiquement le transducteur 107, le circuit électronique 120, et la batterie électrique du dispositif.

Les figures 2A à 2H illustrent de façon schématique et partielle des étapes successives d'un autre mode de réalisation d'un procédé de fabrication d'un dispositif médical implantable comprenant au moins un transducteur ultrasonore.

La figure 2A est une vue en coupe illustrant un substrat 201 de départ un matériau électriquement isolant. L'épaisseur du substrat 201 est par exemple comprise entre 0,2 et 3 mm.

La figure 2B est une vue en coupe illustrant la structure obtenue à l'issue d'une étape de formation d'une ouverture localisée 203 traversant verticalement de part en part le substrat 201. L'ouverture 203 définit un logement destiné à recevoir un transducteur ultrasonore du dispositif. A titre d'exemple, l'ouverture 203 présente des dimensions latérales comprises entre 0,2 et 2 cm, par exemple entre 0,5 et 1 cm. L'ouverture 203 est par exemple formée par ablation laser. En vue de dessus, l'ouverture 203 a par exemple une forme rectangulaire.

La figure 2C est une vue en coupe illustrant la structure obtenue à l'issue d'une étape de fixation du substrat 201, par sa face inférieure, sur la face supérieure d'un deuxième substrat 205, par exemple en un matériau biocompatible. Le substrat 205 est par exemple un substrat métallique. A titre d'exemple, le substrat 205 est en titane. Le substrat 205 s'étend par exemple de façon continue et avec une épaisseur uniforme sur toute la surface inférieure du substrat 201. En particulier, le substrat 205 vient fermer la face inférieure de l'ouverture 203. Ainsi, l'ouverture 203 définit, dans l'empilement comprenant les substrats 201 et 205, une cavité destinée à recevoir un transducteur ultrasonore du dispositif médical. La fixation du substrat 201 sur le substrat 205 est par exemple obtenue par collage. Une couche intermédiaire (non représentée) d'un matériau de collage est par exemple disposée à l'interface entre les deux substrats. L'épaisseur du substrat 201 est par exemple comprise entre 0,2 et 1 mm.

Le substrat 205 est par exemple destiné à former une partie d'un boîtier d'encapsulation du dispositif médical implantable. Dans cet exemple, la face supérieure de l'empilement 205-201 correspond à une face interne du boîtier, destinée à recevoir des composants internes du dispositif médical, et notamment au moins un transducteur ultrasonore. A titre d'exemple, la face inférieure du substrat 205 correspond à une face externe du boîtier, destinée à être mise en contact avec des tissus biologiques d'un patient. L'empilement 205-201 a par exemple la forme générale d'une plaque, par exemple rectangulaire. Les dimensions latérales de la plaque (correspondant aux dimensions latérales du boîtier) sont par exemple comprises entre 1 et 10 centimètres. Sur les figures 2A à 2H, la réalisation d'un unique dispositif médical implantable est (partiellement) représentée.

La figure 2D est une vue de dessus illustrant la structure obtenue à l'issue d'une étape de formation d'éléments métalliques d'interconnexion 211 sur la face supérieure de la structure de la figure 2C, et, plus particulièrement, sur la face supérieure du substrat 201. On notera que les vues en coupe des figures 2A à 2H sont prises dans le plan de coupe C-C de la figure 2D.

A titre d'exemple, une couche métallique s'étendant de façon continue sur toute la surface supérieure de la structure peut d'abord être déposée, cette couche étant ensuite retirée localement, par exemple par photolithographie et gravure, pour définir les éléments d'interconnexion 211. Plus généralement, à cette étape, un ou plusieurs niveaux de circuits imprimés peuvent être formés sur la face supérieure du substrat 201. A titre de variante, une carte de circuit imprimé, par exemple flexible, peut être reportée et fixée sur la face supérieure du substrat 201, à côté de la cavité 203. A titre de variante, le substrat 201 peut être remplacé par une carte de circuit imprimé rigide. A titre de variante, les étapes de la figures 2D peuvent être mises en oeuvre avant l'étape de la figure 2C, avant ou après l'étape de la figure 2B.

La figure 2E est une vue en coupe illustrant la structure obtenue à l'issue d'une étape de report et de fixation d'un transducteur ultrasonore 207 dans la cavité 203.

La figure 2F est une vue de dessus de la structure de la figure 2E.

Le transducteur 207 est par exemple un transducteur piézoélectrique. Dans cet exemple, le transducteur comprend deux électrodes 207a et 207b ramenées du côté de sa face supérieure. Plus particulièrement, dans cet exemple, l'électrode 207a correspond à l'électrode supérieure du transducteur et occupe la majeure partie de la surface supérieure du transducteur. L'électrode 207b ramène, du côté de la face supérieure, le contact électrique de la face inférieure du transducteur. A titre d'exemple, une électrode métallique 206 s'étend du côté de la face inférieure du transducteur, par exemple sur toute la surface inférieure du transducteur ou sur la majeure partie de la surface inférieure du transducteur. Les électrodes 206 et 207a sont par exemple en contact respectivement avec la face inférieure et avec la face supérieure d'une couche piézoélectrique du transducteur. Une ou plusieurs couches intermédiaires (non détaillées sur les figures) de réflexion (miroir acoustique) ou d'absorption des ondes acoustiques, par exemple électriquement conductrices, peuvent éventuellement être prévues entre la face supérieure du matériau piézoélectrique et l'électrode supérieure 207a. L'électrode 207b peut être connectée à l'électrode 206 par l'intermédiaire d'un via électriquement conducteur (non visible sur les figures) traversant verticalement le transducteur ou par l'intermédiaire d'une piste métallique (non visible sur les figures) s'étendant sur un flanc du transducteur. A titre de variante, une métallisation peut être formée au fond de la cavité 203 avant le report du transducteur, permettant de récupérer le contact sur l'électrode inférieure du transducteur et de ramener ce contact, via des pistes conductrices, sur la face supérieure de la structure 201. Comme décrit précédemment, la couche piézoélectrique du transducteur est par exemple en une céramique piézoélectrique, par exemple de type PZT (titano-zirconate de plomb).

Le transducteur 207 est par exemple fixé, par sa face inférieure, sur la face supérieure du substrat 205 au fond de la cavité 203. La fixation s'effectue par exemple à l'aide d'une colle, non visible sur la figure. La colle peut être électriquement conductrice, par exemple lorsque l'on souhaite appliquer le potentiel électrique du substrat 205, en contact avec les tissus biologiques, sur l'électrode inférieure du transducteur. A titre de variante, la colle peut être électriquement isolante.

A titre d'exemple, le transducteur 207 présente des dimensions latérales sensiblement identiques à celles de la cavité 203, par exemple inférieures de moins de dix pourcents aux dimensions latérales de la cavité 203. Ainsi, le transducteur 207 occupe sensiblement toute la surface de la cavité 203. L'épaisseur du transducteur 207 est par exemple sensiblement égale à la profondeur de la cavité 203, par exemple égale, à 20 pourcents près, de préférence à 10 pourcents près, à la profondeur de la cavité 203 (correspondant dans cet exemple à l'épaisseur du substrat 201). Le transducteur 207 peut être prélevé sur un support et placé dans la cavité 203 au moyen d'un outil de prélèvement et placement (« pick and place » en anglais).

On notera que les modes de réalisation décrits ne se limitent pas à l'exemple décrit ci-dessus dans lequel le transducteur 207 est un transducteur piézoélectrique. Plus généralement, les modes de réalisations peuvent être adaptés à tout type de transducteur ultrasonore, par exemple des transducteurs capacitifs à membrane (CMUT), des transducteurs piézoélectriques à membrane (PMUT), etc. Une ou plusieurs couches intermédiaires d'adaptation d'impédance acoustique et/ou d'adaptation de travail de sortie, par exemple électriquement conductrices, peuvent éventuellement être prévues entre la face inférieure du transducteur et le substrat 205. En tout état de cause, selon un aspect du mode de réalisation des figures 2A à 2H, aucune couche d'air, de gaz ou de vide ne sépare la face inférieure du transducteur 207 de la face supérieure du substrat 205, au fond de la cavité 203. Autrement dit, le transducteur 207 est en contact mécaniquement avec la face supérieure du substrat 205, ou séparée de la face supérieure du substrat 205 uniquement par un ou plusieurs matériaux solides. Ceci permet avantageusement de favoriser les transferts d'énergie acoustique entre le transducteur et le milieu extérieur, par l'intermédiaire du substrat 205. Plus particulièrement, dans l'exemple détaillé en relation avec les figures, aucune couche d'air, de gaz ou de vide ne sépare la face inférieure de la couche piézoélectrique du transducteur 207 de la face supérieure du substrat 205, au fond de la cavité. Autrement dit, la couche piézoélectrique du transducteur 207 est en contact mécaniquement avec la face supérieure du substrat 205, ou séparée de la face supérieure du substrat 205 uniquement par un ou plusieurs matériaux solides.

De préférence, l'épaisseur du substrat 201 est choisie de façon à favoriser le couplage acoustique entre le transducteur 207 et le milieu extérieur au boîtier.

Dans cet exemple, les électrodes 207a et 207b du transducteur sont connectées électriquement respectivement à deux plages métalliques de connexion 211 formées sur la face supérieure du substrat 201 (figure 2D) au moyen de fils électriquement conducteurs 209a, 209b, par exemple métalliques, par exemple en or ou en cuivre.

La figure 2G est une vue en coupe illustrant la structure obtenue à l'issue d'une étape de report d'un circuit électronique de contrôle 220 sur la face supérieure de la structure des figures 2E et 2F.

Dans cet exemple, le circuit électronique de contrôle 220 est un circuit intégré dans et sur un substrat semiconducteur, par exemple un substrat de silicium. Le circuit 220 présente des plots de connexion 221 connectés électriquement, par exemple par soudure, à des plages métalliques de connexion correspondante 211 du dispositif. Dans cet exemple, le circuit électronique de contrôle comprend deux plots de connexion connectés respectivement à l'électrode inférieure et à l'électrode supérieure du transducteur 207. Le circuit électronique de contrôle 220 peut en outre comprendre d'autres plots de connexion connectés à des bornes d'alimentation et/ou d'entrée/sortie de signaux de contrôle et/ou de données du dispositif. A titre de variante, le circuit électronique de contrôle 220 peut comprendre plusieurs puces semiconductrices et/ou plusieurs composants électroniques discrets interconnectés entre eux et avec le transducteur ultrasonore 207 par l'intermédiaire d'éléments d'interconnexion formés sur la face supérieure du substrat 201.

Bien que non représentée sur les figures, une batterie électrique peut être reportée sur la face supérieure du substrat 201 et connectée électriquement à des éléments d'interconnexion formés sur la face supérieure du substrat 201. La batterie électrique est par exemple connectée électriquement au circuit électronique de contrôle 220.

La figure 2H est une vue en coupe illustrant la structure obtenue à l'issue d'une étape optionnelle de dépôt d'une couche électriquement isolante de passivation 213 sur la face supérieure de la structure de la figure 2H. La couche 213 est par exemple en un matériau polymère. A titre d'exemple, la couche 213 s'étend de façon continue sur toute la surface supérieure de la structure et recouvre notamment le circuit électronique 220 et le transducteur ultrasonore 207. A titre de variante, la couche 213 peut être retirée au-dessus du transducteur 207 pour ne pas altérer les caractéristiques acoustiques du dispositif. De façon similaire à ce qui a été décrit ci-dessus, à l'issue de ces étapes, un capot supérieur (non détaillé sur les figures) du boîtier du dispositif, de préférence en un matériau biocompatible, par exemple en le même matériau que le substrat 205, peut être rapporté sur la face supérieure de la structure, de façon à encapsuler hermétiquement les composants internes du dispositif.

La figure 3 illustre de façon schématique et partielle une variante du dispositif de la figure 2H.

Le dispositif de la figure 3 diffère du dispositif de la figure 2H principalement en ce qu'il comprend une batterie électrique 301 logée dans une cavité 203' similaire à la cavité 203 (ouverture traversante formée dans le substrat 201) mais présentant des dimensions latérales adaptées aux dimensions de la batterie. A titre d'exemple, en vue de dessus, la batterie présente une surface d'au moins un centimètre carré. La batterie 301 est connectée électriquement à des éléments d'interconnexion formés sur la face supérieure du substrat 201. La batterie électrique est par exemple connectée électriquement au circuit électronique de contrôle 220.

Dans l'exemple représenté, la batterie 301 comprend une électrode supérieure connectée électriquement à une plage métallique de connexion 211 disposée sur la face supérieure du substrat 201 au moyen d'un fil conducteur 303, par exemple un fil métallique, par exemple en cuivre, et une électrode inférieure en contact mécaniquement et électriquement avec la face supérieure du substrat métallique 205. Dans cet exemple, le substrat métallique 205 sert de plan de masse. Un via électriquement conducteur 305 traversant verticalement le substrat 201 connecte électriquement le substrat 205 à un élément métallique de connexion 211 disposé sur la face supérieure du substrat 201.

On notera que de façon similaire, dans l'exemple des figures 2A à 2H, une électrode inférieure du transducteur 207 peut être connectée électriquement à un élément métallique d'interconnexion 211 par l'intermédiaire du substrat métallique 205 et d'un via électriquement conducteur traversant verticalement de part en part le substrat 201.

A titre de variante, dans l'exemple de la figure 3, les deux électrodes de la batterie 301 sont ramenées en face supérieure et sont connectées à des éléments de connexion métallique 211 disposés sur la face supérieure du substrat 201 par respectivement deux fils conducteurs.

Un avantage des modes de réalisation décrits est qu'ils permettent une intégration compacte et performante de transducteurs ultrasonores dans un dispositif médical implantable, par exemple, en vue de permettre un transfert d'énergie électrique sans fil par ultrasons vers le dispositif médicale implantable.

Divers modes de réalisation et variantes ont été décrits. La personne du métier comprendra que certaines caractéristiques de ces divers modes de réalisation et variantes pourraient être combinées, et d'autres variantes apparaîtront à la personne du métier.

Enfin, la mise en oeuvre pratique des modes de réalisation et variantes décrits est à la portée de la personne du métier à partir des indications fonctionnelles données ci-dessus.

## Revendications

1. Dispositif médical implantable comportant un premier substrat (101 ; 205) en un matériau biocompatible, le premier substrat (101 ; 205) définissant une partie d'un boîtier du dispositif et présentant une face externe destinée à être mise en contact avec des tissus biologiques d'un utilisateur, le dispositif comportant au moins un transducteur ultrasonore (107 ; 207) disposé dans une cavité (103 ; 203) formée du côté d'une face interne du premier substrat (101 ; 205), le transducteur (107 ; 207) étant agencé de sorte qu'aucune couche d'air, de gaz ou de vide ne sépare le transducteur (107) du premier substrat (101 ; 205).

2. Dispositif selon la revendication 1, dans lequel la cavité (103) est une cavité non traversante formée dans le premier substrat (101), du côté de la face interne du premier substrat (101).

3. Dispositif selon la revendication 2, dans lequel le premier substrat (101) est en un matériau électriquement isolant, par exemple du saphir.

4. Dispositif selon la revendication 1, comprenant en outre un deuxième substrat (201) fixé sur le premier substrat (205), du côté de la face interne du premier substrat (205), la cavité (203) étant une cavité traversante formée dans le deuxième substrat (201) et débouchant sur le premier substrat (205).

5. Dispositif selon la revendication 4, dans lequel le premier substrat (205) est en métal, par exemple en titane, le deuxième substrat (201) étant en un matériau électriquement isolant ou comprenant une carte de circuit imprimé.

6. Dispositif selon l'une quelconque des revendications 1 à 5, comportant en outre, au-dessus de la face supérieure du premier substrat (101 ; 205), des éléments métalliques d'interconnexion connectés respectivement à des première (105 ; 207a) et deuxième (107b ; 207b) électrodes du transducteur (107 ; 207).

7. Dispositif selon la revendication 6, comportant en outre, au-dessus de la face supérieure du premier substrat (101 ; 205), un circuit électronique de contrôle (120 ; 220) connecté aux première (105 ; 207a) et deuxième (107b ; 207b) électrodes du transducteur (107 ; 207) par l'intermédiaire desdits éléments métalliques d'interconnexion.

8. Dispositif selon la revendication 7, comportant en outre une batterie électrique (301) au-dessus de la face supérieure du premier substrat et connecté au circuit électronique de contrôle (120 ; 220).

9. Dispositif selon la revendication 8, dans lequel la batterie (301) est disposée dans une cavité formée du côté de la face interne du premier substrat (101 ; 205), à l'intérieur du boîtier.

10. Dispositif selon la revendication 8 ou 9, dans lequel le circuit électronique de contrôle (120 ; 220) est adapté à convertir une puissance électrique fournie par le transducteur ultrasonore (107 ; 207) en un signal électrique de recharge de la batterie (301) et/ou d'alimentation électrique de composants électroniques du dispositif.

11. Dispositif selon l'une quelconque des- revendications 1 à 10, dans lequel le transducteur (107 ; 207) comprend une couche en un matériau piézoélectrique, le transducteur (107 ; 207) étant agencé de sorte qu'aucune couche d'air, de gaz ou de vide ne soit présente entre ladite couche piézoélectrique et le premier substrat (101 ; 205).

12. Dispositif selon la revendication 11, dans lequel la couche piézoélectrique du transducteur (107 ; 207) est en une céramique piézoélectrique, par exemple du PZT.

13. Dispositif selon l'une quelconque des revendications 1 à 12, comprenant en outre un capot supérieur en le même matériau que le premier substrat (101 ; 205), par exemple soudé au premier substrat (101 ; 205), encapsulant hermétiquement des composants internes du dispositif.

14. Procédé de fabrication d'un dispositif selon l'une quelconque des revendications 1 à 13, comprenant une étape de formation de ladite cavité (103 ; 203), suivie d'une étape de report et de fixation du transducteur (107 ; 207) dans la cavité.

15. Procédé selon la revendication 14, dans lequel la cavité est formée par ablation laser.
